# EUROPEAN PATENT SPECIFICATION

(11) **EP 2 534 998 B1**
(45) Date of publication and mention of the grant of the patent: **31.12.2014**
(21) Application number: 12172025.4
(22) Date of filing: 14.06.2012
(51) Int. Cl.: A61B 1/015, A61B 1/00

(54) **Insertion assisting tool for endoscope**
Einführhilfswerkzeug für Endoskop
Outil d'aide à l'insertion d'un endoscope

(30) Priority: 16.06.2011 JP 2011134488
(43) Date of publication of application: 19.12.2012
(73) Proprietor: Fujifilm Corporation, Minato-ku Tokyo 106-8620 (JP)
(72) Inventor: Iyama, Shozo, Ashigarakami-gun, Kanagawa, 258-8538 (JP); Ohki, Tomohiro, Ashigarakami-gun, Kanagawa, 258-8538 (JP); Inoue, Masaya, Ashigarakami-gun, Kanagawa, 258-8538 (JP); Iwasaka, Masayuki, Ashigarakami-gun, Kanagawa, 258-8538 (JP)
(74) Representative: Klunker . Schmitt-Nilson . Hirsch

(56) References cited:
- EP-A2- 1 559 361
- DE-A1- 19 714 950
- JP-A- 60 185 532

## Description

### BACKGROUND OF THE INVENTION

### Field of the Invention

The present invention relates to an insertion assisting tool for an endoscope, and in particular to an insertion assisting tool for an endoscope used in inserting an insertion part of an endoscope into a biliary tract or a pancreatic duct.

### Description of the Related Art

In recent years, endoscopic examination or treatment of pancreaticobiliary diseases, such as biliary tract cancer, pancreas cancer, cholelithiasis, and choledocholithiasis, has been widely spreading in the field of medicine. These endoscopic examination and treatment have the advantages of being less invasive and imposing less strain on patients than conventional surgical treatment.

As a method for these examination and treatment, for example, ERCP (Endoscopic Retrograde Cholangio-Pancreatography) is known. The ERCP is a diagnosis method using an endoscope to inject contrast medium into the biliary tract or the pancreatic duct and photograph the injected region with a fluoroscope. In the method of injecting the contrast medium, first, an insertion part of the endoscope is inserted until a distal end portion of the insertion part reaches the duodenum. Then, a cannula (thin tube) is fed from a forceps exit provided at the distal end portion of the insertion part, the cannula is inserted from the major duodenal papilla selectively into the biliary tract or the pancreatic duct, a contrast medium is injected into the biliary tract or the pancreatic duct through the cannula, and the injected portion is photographed by a fluoroscope.

A method of confirming the presence or absence of a site of stenosis inside the biliary tract or the pancreatic duct, sampling and examining cells or tissues (cytodiagnosis, biopsy), crushing and removing calculi, or the like by inserting the insertion part of a thin endoscope generally called cholangioscope or pancreatoscope into the biliary tract or the pancreatic duct is also known.

Under these circumstances, when the insertion part of the endoscope is inserted into a body cavity, an insertion assisting tool (also called "overtube" or "sliding tube") for an endoscope is used together. As one example of the insertion assisting tool for an endoscope, an insertion assisting tool for an endoscope including a tubular body used as a guide through which the insertion part of the endoscope is passed, a distal sidewall portion of the tubular body being provided with an opening part (referred to as sidewall opening part below) from which a distal end of the insertion part can be fed is disclosed in Japanese Patent Application Laid-Open No. S60-185532. According to this insertion assisting tool for an endoscope, when the insertion part of the endoscope is inserted into a body cavity, it is made possible by inserting the insertion part covered with the tubular body to perform easy insertion of the insertion part while preventing undesired bending or flexing of the insertion part. Further, when the distal end of the insertion part is fed from the sidewall opening part of the tubular body and is guided into a body cavity (for example, the biliary duct), the insertion part can be easily inserted further into a deep portion of the body cavity by inserting the distal end of the insertion part while receiving and supporting the insertion part by an edge of the sidewall opening part.

Further, an insertion assisting tool for an endoscope, which has an outer circumferential face provided with a lubricating liquid supply passage and which is formed with a plurality of openings for supplying lubricating liquid injected to the lubricating liquid supply passage inside the insertion assisting tool at predetermined intervals is disclosed in Japanese Patent Application Laid-Open No. 2005-237947. According to this insertion assisting tool for an endoscope, lubricating liquid can be supplied evenly over a whole area of the inner circumferential face of the insertion assisting tool without enlarging the diameter of the insertion assisting tool.

### SUMMARY OF THE INVENTION

In the conventional insertion assisting tool for an endoscope disclosed in the Japanese Patent Application Laid-Open No. S60-185532, however, when the distal end of the insertion part is fed from the sidewall opening part of the tubular body to be guided into a body cavity, the distal end of the insertion part is inserted while the insertion part is received and supported by an edge of the sidewall opening part, which results in such a problem that operability of the insertion part deteriorates due to frictional resistance occurring between the insertion part and the edge of the sidewall opening part.

By supplying lubricating liquid to a whole area of the inner circumferential face of the insertion assisting tool against such a problem like the insertion assisting tool for an endoscope disclosed in the Japanese Patent Application Laid-Open No. 2005-237947, a sliding performance of the insertion part of the endoscope when the insertion operation such as described in the Japanese Patent Application Laid-Open No. S60-185532 is performed can be improved to some extent, but a sufficient effect of the sliding performance cannot be obtained.

The present invention has been made in view of these circumstances, and an object of the present invention is to provide an insertion assisting tool for an endoscope where operability of an insertion part of an endoscope when the insertion part is inserted into a body cavity has been improved.

In order to achieve the above object, according to an aspect of the present invention, there is provided an insertion assisting tool for an endoscope including: a tubular body having a passage through which an insertion part of an endoscope is inserted; a sidewall opening part which is provided in a sidewall part of the tubular body on a distal end side of the tubular body and through which a distal end part of the insertion part can be fed, the sidewall opening part having an edge part receiving and supporting the insertion part when the distal end part of the insertion part is fed from the sidewall opening part to be guided into a body cavity; a supply port formed at the edge part of the sidewall opening part; and a lubricating liquid supply passage which is in communication with the supply port to supply lubricating liquid to the supply port.

According to the aspect of the present invention, since lubricating liquid is supplied to the edge part of the sidewall opening part, when the insertion part of the endoscope is inserted into a body cavity while being received and supported by the edge portion of the sidewall opening part, frictional resistance occurring between the insertion part and the edge portion of the sidewall opening part can be reduced. Thereby, the operability of the insertion part of the endoscope can be improved.

In another aspect of the present invention, it is preferred that the edge portion of the sidewall opening part is positioned on the distal end side of the sidewall opening part. According to the aspect of the present invention, when the insertion part of the endoscope is inserted into a body cavity while being received and supported by the edge portion of the sidewall opening part, frictional resistance occurring between the insertion part and the edge portion of the sidewall opening part can be reduced.

In still another aspect of the present invention, it is preferred that the lubricating liquid supply passage is provided inside a sidewall part of the tubular body. According to the aspect of the present invention, since a diameter reduction of the tubular body can be achieved by utilizing an internal space of the sidewall part of the tubular body, burden of a patient can be reduced.

In another aspect of the present invention, it is preferred that the supply port is composed of a plurality of supply port elements arranged at the edge portion of the sidewall opening part. According to the aspect of the present invention, stable supply of lubricating liquid is made possible.

In another aspect of the present invention, it is preferred that a porous body is provided at the edge portion of the sidewall opening part and the supply port is composed of fine holes formed on a surface of the porous body. According to the aspect of the present invention, since it is made possible to supply lubricating liquid from the fine hole formed on the surface of the porous body at a constant flow rate at a low speed and frictional resistance occurring between the insertion part and the edge portion of the sidewall opening part can be further effectively reduced while preventing lubricating liquid from scattering around the sidewall opening part.

In still another aspect of the present invention, it is preferred that a corner portion of the edge part of the sidewall opening part is chamfered. According to the aspect of the present invention, lubricating liquid is prevented from being scrapped off due to contact of the insertion part with the corner portion of the edge portion of the sidewall opening part occurring when insertion is performed while the insertion part is being received and supported by the sidewall opening part of the tubular body.

According to the present invention, since lubricating liquid is supplied to the edge portion of the sidewall opening part of the tubular body, frictional resistance occurring between the insertion part of the endoscope and the edge portion of the sidewall opening part can be made small when the insertion part of the endoscope is inserted into a body cavity while being received and supported by the edge portion of the sidewall opening part. Thereby, operability of the insertion part of the endoscope can be improved.

### BRIEF DESCRIPTION OF THE DRAWINGS

Fig. 1 is an appearance view showing an endoscopic device applied with an insertion assisting tool for an endoscope according to a first embodiment;
Fig. 2 is a perspective view showing a configuration example of the vicinity of a distal end of an insertion part of an endoscope;
Fig. 3 is a schematic view (plan view) showing a configuration example of the vicinity of the distal end of the insertion assisting tool;
Fig. 4 is a schematic view (side sectional view) showing a configuration example of the vicinity of the distal end of the insertion assisting tool;
Fig. 5 is a schematic view (front sectional view) showing a configuration example of the vicinity of the distal end of the insertion assisting tool;
Fig. 6 is an explanatory view showing a procedure for inserting the insertion part of the endoscope into a bilinary tract through the use of the insertion assisting tool;
Fig. 7 is an explanatory view showing a procedure for inserting the insertion part of the endoscope into the bilinary tract through the use of the insertion assisting tool;
Fig. 8 is an explanatory view showing a procedure for inserting the insertion part of the endoscope into the bilinary tract through the use of the insertion assisting tool;
Fig. 9 is a plan view showing a configuration example of the vicinity of a distal end of an insertion assisting tool according to a second embodiment;
Fig. 10 is a front sectional view showing a configuration example of the vicinity of the distal end of the insertion assisting tool according to the second embodiment;
Fig. 11 is a plan view showing a configuration example of the vicinity of a distal end of an insertion assisting tool according to a third embodiment;
Fig. 12 is a plan view showing a configuration example of the vicinity of a distal end of an insertion assisting tool according to a fourth embodiment;
Fig. 13 is a front sectional view showing a configuration example of the vicinity of the distal end of the insertion assisting tool according to the fourth embodiment;
Fig. 14 is a side sectional view showing a configuration example of the vicinity of a distal end of an insertion assisting tool according to a fifth embodiment;
Fig. 15 is a side sectional view showing a configuration example of the vicinity of a distal end of an insertion assisting tool according to a sixth embodiment;
Fig. 16 is a side sectional view showing a configuration example of the vicinity of a distal end of an insertion assisting tool according to a seventh embodiment;
Fig. 17 is a side sectional view showing a configuration example of a distal end side of an insertion assisting tool according to an eighth embodiment;
Fig. 18 is a side sectional view showing a configuration example of a proximal end side of an insertion assisting tool according to the eighth embodiment; and
Fig. 19 is a perspective view showing a piece member provided in an insertion assisting tool according to the eighth embodiment.

### DETAILED DESCRIPTION OF THE PREFERRED EMBODIMENTS

Preferred embodiments of the present invention will be described below in detail with reference to the accompanying drawings.

### <First Embodiment>

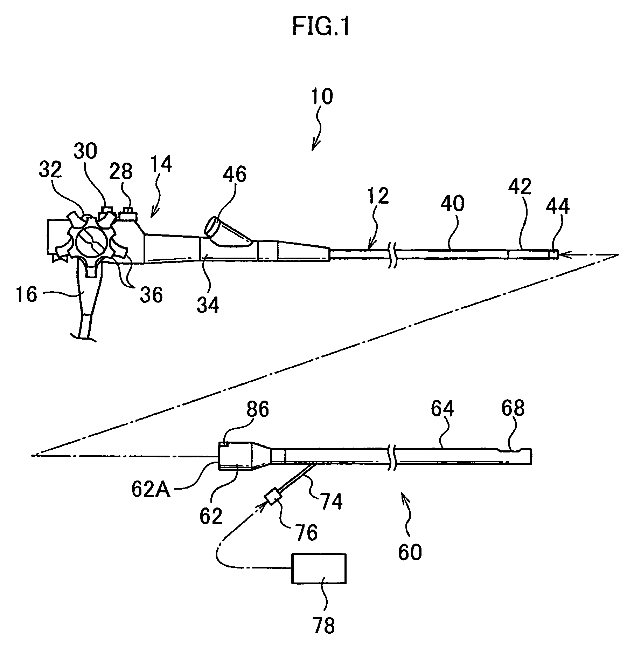

Fig. 1 is an appearance view showing an endoscope apparatus to which an insertion assisting tool for an endoscope (simply referred to as "insertion assisting tool" below) 60 according to an embodiment of the present invention has been applied. As shown in Fig. 1, an endoscopic device is mainly composed of an endoscope 10 and an insertion assisting tool 60.

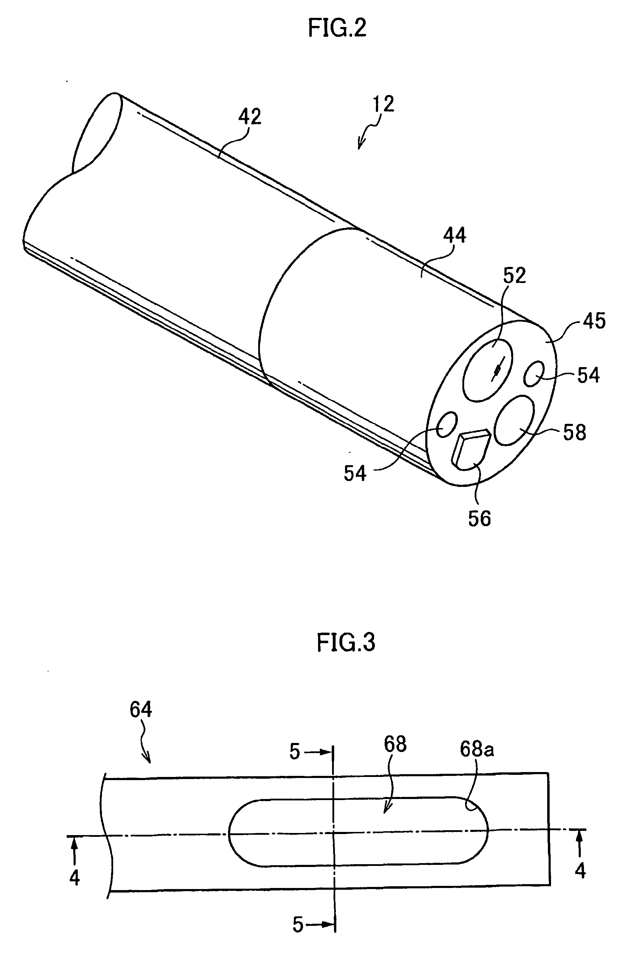

The endoscope 10 is provided with a hand operation part 14 and an insertion part 12 provided to be connected to this hand operation part 14 and inserted into a body of a human. The hand operation part 14 is connected with a universal cable 16 and a distal end of the universal cable 16 is provided with a light guide (LG) connector. The LG connector is attachably and detachably coupled with a light source device not shown in the figures by which illumination light is transmitted to an illumination optical system (illumination window) 54 (see Fig. 2) described later. The LG connector is also connected with an electric connector, and this electric connector is attachably and detachably coupled with a processor that performs image signal processing or the like.

The hand operation part 14 is provided with a gas-supply/water-supply button 28, a suction button 30 and a shutter button 32 positioned side by side and also provided with a pair of angle knobs 36 and 36.

The insertion part 12 is composed of a flexible part 40, a bending part 42 and a distal end portion 44 in this order from the hand operation part 14, and the bending part 42 is bend remotely by rotating the angle knobs 36 and 36 of the hand operation part 14. This makes it possible to turn the distal end portion 44 to a desired direction.

As shown in FIG. 2, a distal end face 45 of the distal end portion 44 is provided with an observation optical system (observation window) 52, an illumination optical systems (illumination windows) 54 and 54, a gas-supply/water-supply nozzle 56, and a forceps channel 58. An imaging device (not shown) such as CCD or CMOS is disposed behind the observation optical system 52, and a substrate for supporting the imaging device is connected with a signal cable (not shown). The signal cable is extended to the electric connector through the insertion part 12, the hand operation part 14, and the universal cable 16, and the like, which are shown in Fig. 1, to be connected to the processor. Therefore, an observation image captured by the observation optical system 52 is imaged on a light-receiving face of the imaging device and converted into an electric signal, and the electric signal is then outputted to the processor through the signal cable, and converted into a video signal. Thereby, the observation image is displayed on a monitor connected to the processor.

A light emission end of the light guide (not shown) is disposed behind the illumination optical systems 54 and 54 shown in Fig. 2. The light guide is extended through the insertion part 12, the hand operation part 14, and the universal cable 16 which are shown in Fig. 1, and an incident end of the light guide is disposed within the LG connector. Therefore, by coupling the LG connector to the light source device, illumination light emitted from the light source device is transmitted to the illumination optical systems 54 and 54 through the light guide, and emitted forward from the illumination optical systems 54 and 54.

The gas-supply/water-supply nozzle 56 shown in Fig. 2 is in communication with a valve (not shown) operated by the gas-supply/water-supply button 28 shown in Fig. 1, and the valve is further in communication with a gas-supply/water-supply connector (not shown) provided in the LG connector. A gas-supply/water-supply device (not shown) is connected to the gas-supply/water supply connector to feed air and water to the gas-supply/water supply connector. By operating the gas-supply/water-supply button 28, air or water can be jetted from the gas-supply/water-supply nozzle 56 toward the observation optical system 52.

The forceps opening 58 shown in Fig. 2 is in communication with the forceps entrance 46 shown in Fig. 1 through a forceps channel (not shown). Therefore, by inserting a treatment instrument such as a forceps from the forceps entrance 46, the treatment instrument can be fed from the forceps opening 58. Further, the forceps opening 58 is in communication with a valve (not shown) operated by the suction button 30, and the valve is further connected to a suction connector (not shown) of the LG connector. Therefore, by connecting a suction device (not shown) to the suction connector and operating the valve by the suction button 30, dirt, residue and the like can be sucked from the forceps opening 58.

On the other hand, the insertion assisting tool 60 shown in Fig. 1 is composed of a tube main body 64 and a grasping part 62. The tube main body 64 is formed in a cylindrical shape and has an inner diameter slightly larger than an outer diameter of the insertion part 12. Further, the tube main body 64 is a flexible molded product made of urethane resin or silicon resin, an outer peripheral surface of the tube main body 64 is covered with a lubricating coat, and an inner peripheral surface of the tube main body 64 is also covered with a lubricating coat. The grasping part 62 which is hard and shown in Fig, 1 is water-tightly fitted to the tube main body 64 so that the grasping part 62 is attachably and detachably or integrally coupled to the tube main body 64. Incidentally, the insertion part 12 is inserted from a proximal end opening part 62A of the grasping part 62 toward the tube main body 64.

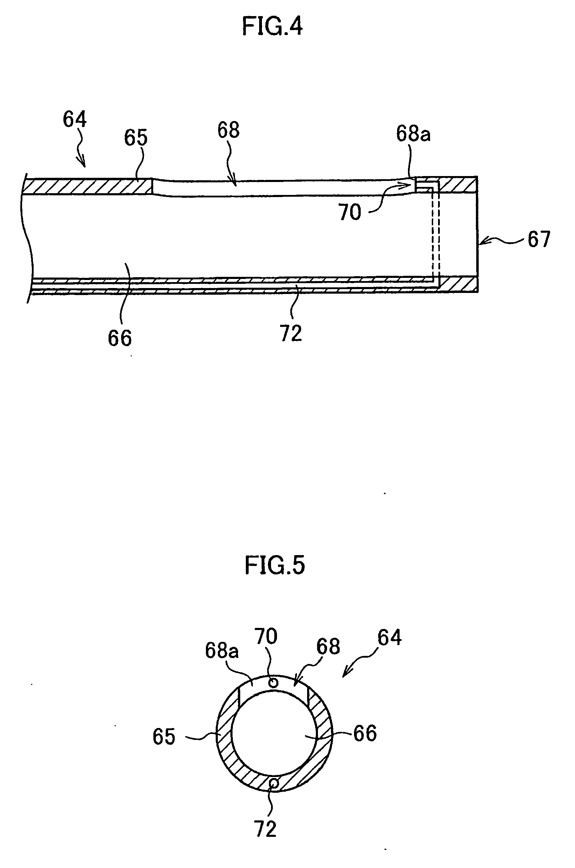

Here, the configuration of the tube main body 64 will be described in detail. Fig. 3 to Fig. 5 are schematic views showing an configuration example of the vicinity of a distal end of the tube main body 64, Fig. 3 being a plan view, Fig. 4 being a side sectional view (a sectional view taken along line 4 - 4 in Fig. 3), and Fig. 5 being a front sectional view (a sectional view taken along line 5 - 5 in Fig. 3).

As shown in Fig. 3 to Fig. 5, an opening part (hereinafter, called "distal end opening part") 67 is formed at a distal end of the tube main body 64, and an insertion passage 66 communicating with the distal end opening part 67 is provided inside the tube main body 64 so as to extend along an axial direction of the tube main body 64. The insertion passage 66 is a channel through which the insertion part 12 (see Fig. 1) of the endoscope 10 is inserted, and is substantially circular in cross section perpendicular to the axial direction.

A sidewall portion 65 of the tube main body 64 on a distal end side of the tube main body 64 is provided with an opening part (hereinafter, called "sidewall opening part") 68 composed of a through-hole formed into a long hole whose longitudinal direction corresponds to the axial direction of the tube main body 64. The sidewall opening part 68 is a hole part from which a distal end of the insertion part 12 inserted into the insertion passage 66 can be fed, an opening width (a length in a direction perpendicular to the axial direction of the tube main body 64) of the sidewall opening part 68 is slightly larger than an outer diameter (diameter) of the insertion part 12 of the endoscope 10, and a length (a length in the axial direction of the tube main body 64) of the sidewall opening part 68 is formed to be sufficiently larger than the opening width. Thereby, as described later, by operating the insertion part 12 inserted in the insertion passage 66 of the tube main body 64 in a bending manner, the distal end of the insertion part 12 can be fed out of the sidewall opening part 68 of the tube main body 64. Incidentally, the grasping part 62 is provided with an index 86 indicating a direction of the sidewall opening part 68.

As shown in Fig. 4 and Fig. 5, an edge part (hereinafter, called "distal end edge part") 68a of the sidewall opening part 68 on the distal end side of the sidewall opening part 68 is provided with a supply port 70 for lubricating liquid. The supply port 70 is in communication with a lubricating liquid supply passage 72. The lubricating liquid supply passage 72 is a channel formed inside the sidewall part 65 of the tube main body 64, where the lubricating liquid supply passage 72 extends from a proximal end side of the tube main body 64 to a distal end side of the tube main body 64 along the axial direction of the tube main body 64, and is connected to the supply port 70 at the distal end side after being turned from the circumferential direction back to the proximal end side.

The lubricating liquid supply passage 72 at the proximal end side is connected to a tube 74 with a small diameter shown in Fig. 1, and a lubricating liquid supply part 78 is connected to the lubricating liquid supply passage 72 via a connector 76 provided at a distal end of the tube 74. The lubricating liquid supply part 78 is composed of, for example, a syringe or the like, and it injects lubricating liquid such as water into the connector 76. Thereby, lubricating liquid injected from the lubricating liquid supply part 78 to the connector 76 is fed from the supply port 70 formed at the distal end edge part 68a of the sidewall opening part 68 through the lubricating liquid supply passage 72.

Incidentally, the lubricating liquid supply passage 72 may be composed of a tube-like member arranged along an outer circumferential face or an inner circumferential face of the tube main body 64. However, by adopting such a configuration that the lubricating liquid supply passage 72 is formed in the sidewall part 65 of the tube main body 64 like this embodiment, diameter reduction of the tube main body 64 can be achieved by utilizing an inner space of the sidewall part of the tube main body 64.

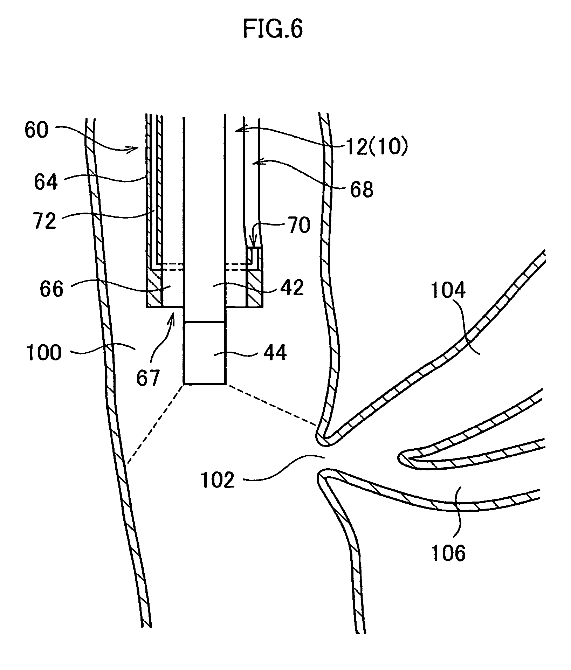

Next, the method of operating the endoscopic device thus configured will be described with reference to Fig. 6 to Fig. 8. Thought a case where the insertion part 12 of the endoscope 10 is inserted into a biliary tract 104 will be herein described as one example, the method is also applied to a case where the insertion part 12 is inserted into a pancreatic duct 106.

First, the insertion part 12 of the endoscope 10 is covered with the insertion assisting tool 60, the insertion part 12 is caused to pass through the insertion passage 66 of the tube main body 64, as shown in Fig. 6, and in this state the insertion part 12 and the tube main body 64 are inserted through a patient's mouth such that the distal end of the insertion part 12 is positioned in a duodenum 100 through the stomach. At this time, the distal end of the insertion part 12 is fed from the distal opening part 67 such that an observation direction (direction of observational field of view) of the observation optical system 52 of the insertion part 12 substantially corresponds to the axial direction of the tube main body 64.

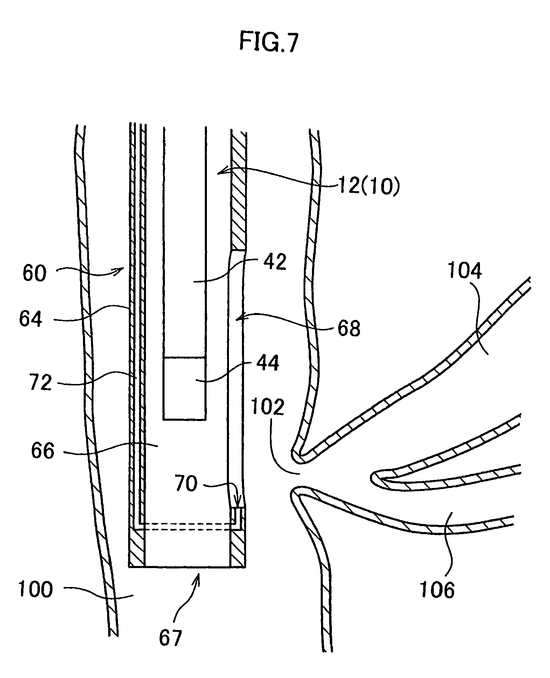

Next, after a major duodenal papilla 102 is confirmed through an observation image observed by the observation optical system 52 of the insertion part 12, the insertion assisting tool 60 is inserted further forward into a deep portion (distal end) of the duodenum 100, such that the sidewall opening part 68 of the tube main body 64 is positioned to face the major duodenal papilla 102, as shown in Fig. 7.

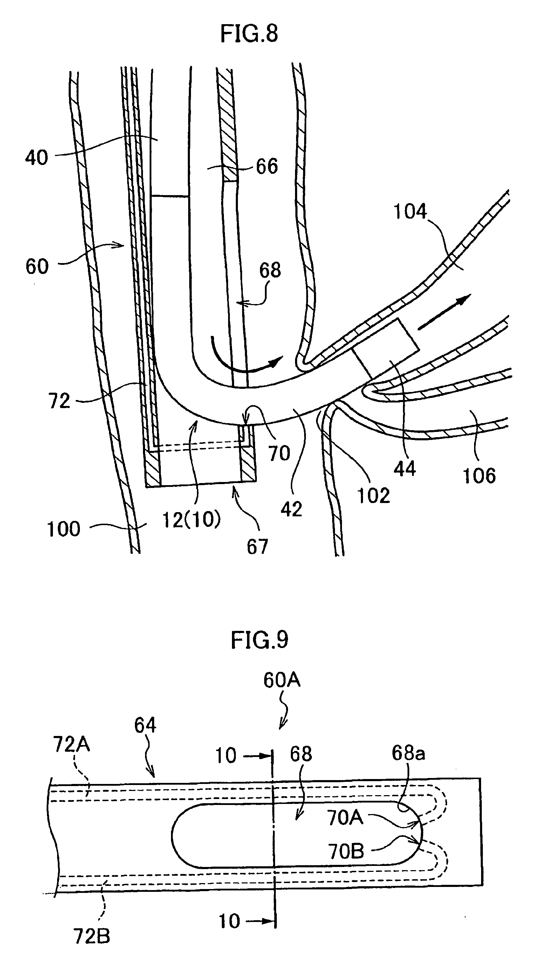

Next, the insertion part 12 is advanced while being operated in a bending manner, the distal end of the insertion part 12 is fed from the sidewall opening part 68 of the tube main body 64 to be inserted from the major duodenal papilla 102 into the biliary tract 104, as shown in Fig. 8. At this time, the distal end of the insertion part 12 can be inserted easily into a deep portion within the biliary tract 104 by inserting and operating the insertion part 12 fed from the sidewall opening part 68 in a bending manner so as to push in the insertion part 12 while receiving and supporting the insertion part 12 by the distal end edge part 68a of the sidewall opening part 68.

While such an operation is being performed, lubricating liquid is supplied to the sidewall opening part 68 of the insertion assisting tool 60. The lubricating liquid is injected from the lubricating liquid supply part 78 shown in Fig. 1 to the connector 76 and is supplied from the supply port 70 provided at the distal end edge part 68a of the sidewall opening part 68 through the lubricating liquid supply passage 72 provided in the sidewall part of the tube main body 64. The distal end edge part 68a of the sidewall opening part 68 is a portion receiving and supporting the insertion part 12, and frictional resistance occurring between the insertion part 12 and the portion can be reduced by supplying lubricating liquid to the portion.

As described above, according to the insertion assisting tool 60 of the present embodiment, since lubricating liquid is supplied to the distal end edge part 68a of the sidewall opening part 68, when the insertion part 12 is guided into a body cavity while being received and supported by the distal end edge part 68a of the sidewall opening part 68, excellent sliding property can be always obtained and slidability of the insertion part 12 to the sidewall opening part 68 of the tube main body 64 is improved, so that the insertion part 12 can be easily inserted into a biliary tract or a pancreatic duct. Thereby, operation time can be shortened and burden on a patient can be reduced.

### <Second Embodiment>

Next, a second embodiment of the present invention will be described. Explanation of parts in common with those in the first embodiment is omitted and feature parts of the present embodiment will be mainly described below.

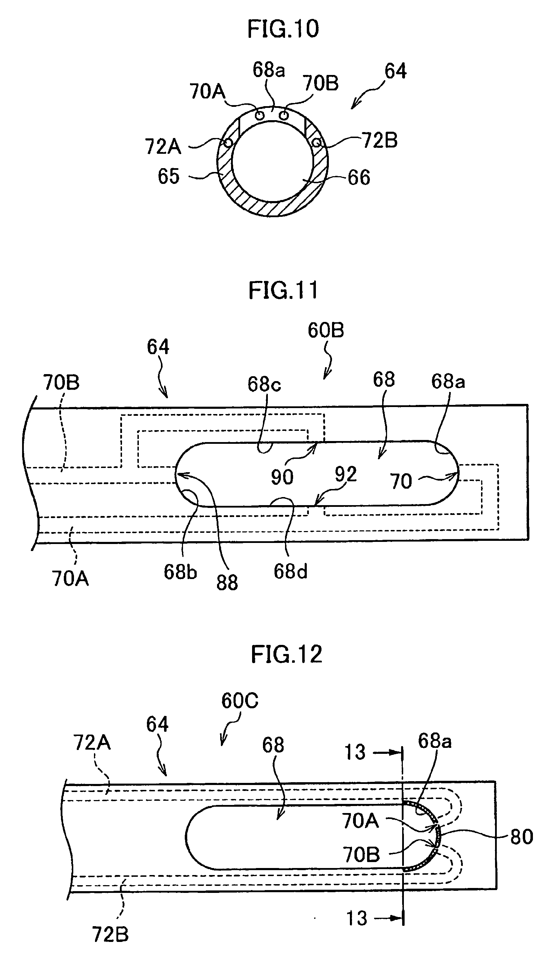

Fig. 9 and Fig. 10 are schematic views showing a configuration example of the vicinity of a distal end of an insertion assisting tool 60A according to the second embodiment, Fig. 9 being a plan view and Fig. 10 being a front sectional view (a sectional view taken along line 10 -10 in Fig. 9).

In the second embodiment, as shown in Fig. 9 and Fig. 10, two supply ports 70A and 70B are provided at the distal end edge part 68a of the sidewall opening part 68 of the tube main body 64, and lubricating liquid supply passages 72A and 72B connected to the respective supply ports 70a and 70B, respectively, are formed in the sidewall part 65 of the tube main body 64. The lubricating liquid supply passages 72A and 72B may be connected to a common supply passage (not shown) on a proximal end side of the tube main body 64, and the lubricating liquid supply passages 72A and 72B may be connected to the lubricating liquid supply part 78 shown in Fig. 1 individually via tubes and connectors provided in the respective lubricating liquid supply passages 72A and 72B.

According to the second embodiment, lubricating liquid can be supplied from a plurality of supply ports 70A and 70B stably, so that frictional resistance occurring between the insertion part 12 and the sidewall opening part 68 can be reduced effectively. Further, even if clogging occurs in one of the plurality of supply ports 70A and 70B, lubricating liquid can be supplied form the other supply port, so that operability of the insertion part 12 can be maintained stably.

Incidentally, in the second embodiment, the configuration where two supply ports 70A and 70B are provided at the distal end edge part 68a of the sidewall opening part 68, but the number of support supply ports is not limited to a specific one, and at least three support ports may be provided.

Further, in the second embodiment, the configuration where the lubricating liquid supply passages 72A and 72B are provided at two supply ports 70A and 70B, respectively, but the present invention is not limited to this configuration, and such a configuration that lubricating liquid is distributed from one lubricating supply passage to a plurality of supply ports may be adopted.

### <Third Embodiment>

Next, a third embodiment of the present invention will be described. Explanation of parts in common with those in the first embodiment is omitted and feature parts of the present embodiment will be mainly described below.

Fig. 11 is schematic view (plan view) showing a configuration example of the vicinity of a distal end of an insertion assisting tool 60B according to the third embodiment.

In the third embodiment, as shown in Fig. 11, the point that the supply port 70 is provided at the distal end edge part 68a of the sidewall opening part 68 of the tube main body 64 is similar to that in the first embodiment. Further, in the third embodiment, a supply port 88 is provided at a rear end edge part 68b of the sidewall opening part 68 and supply ports 90 and 92 are provided at side end parts 68c and 68d on both sides of the sidewall opening part 68, respectively. Further, two lubricating liquid supply passages 70A and 70B are provided in the sidewall part 65 of the tube main body 64. The lubricating liquid supply passage 70A is in communication with the supply ports 70 and 92, and the lubricating liquid supply passage 70B is communication with the supply ports 88 and 90.

According to the third embodiment, even if the rear end edge part 68b, or the side end part 68c or 68d of the sidewall opening part 68 is positioned at a portion for receiving and supporting the insertion part 12 due to change of a relative positional relationship between the insertion assisting tool 60B and the insertion part 12, lubricating liquid is supplied to the portion, so that frictional resistance between the insertion part 12 and the portion can be reduced effectively.

Incidentally, in the third embod9iment, the configuration where two lubricating liquid supply passages 70A and 70B are provided, but the present invention is not limited to this configuration and distribution supply may be performed from one lubricating liquid supply passage to the respective supply ports or lubricating liquid supply passages may be provided corresponding to the respective supply ports.

### <Fourth Embodiment>

Next, a fourth embodiment of the present invention will be described. Explanation of parts in common with those in the first embodiment is omitted and feature parts of the present embodiment will be mainly described below.

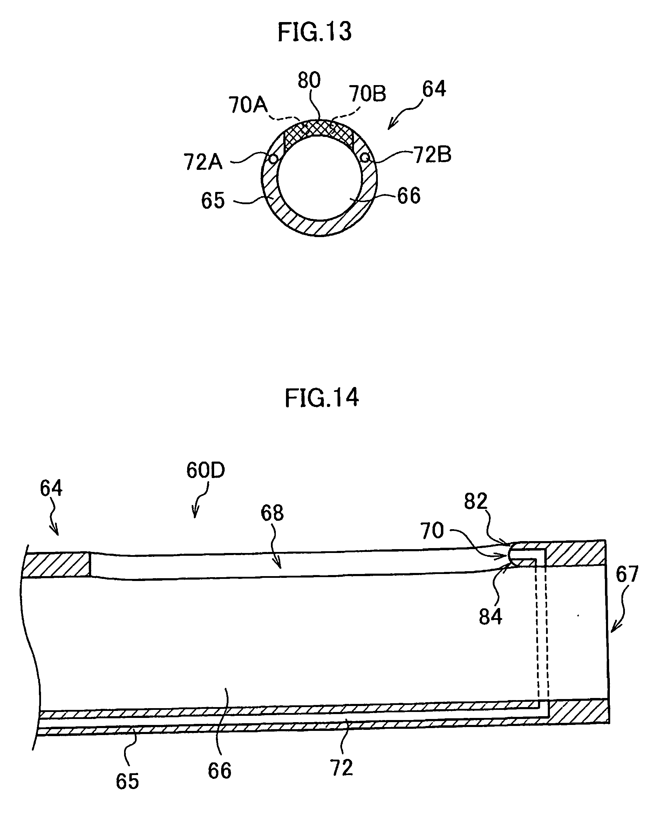

Fig. 12 and Fig. 13 are schematic views showing a configuration example of the vicinity of a distal end of an insertion assisting tool for an endoscope 60C according to the fourth embodiment, Fig. 12 being a plan view and Fig. 13 being a front sectional view (a sectional view taken along line 13 - 13 in Fig. 12).

In the fourth embodiment, as shown in Fig. 12 and Fig. 13, the distal end edge part 68a of the sidewall opening part 68 of the tube main body 64 is composed of a hard porous body 80 and lubricating liquid is supplied from the supply ports 70A and 70B through the porous body 80. As the hard porous body 80, for example, a porous ceramics or the like can be used.

According to the fourth embodiment, since lubricating liquid is supplied at a constant flow rate from fine holes formed on a surface of the porous body 80 due to capillary action of the porous body 80, lubricating liquid is prevented from being scattered around the sidewall opening part 68 and the distal end edge part 68a can be always put in a wet state by lubricating liquid, so that frictional resistance between the insertion part 12 and the sidewall opening part 68 can be reduced effectively.

Incidentally, in the fourth embodiment, only the distal end edge part 68a of the sidewall opening part 68 is composed of the porous body 80, but the present invention may not be limited to this configuration and the whole inner wall face of the sidewall opening part 68 may be composed of the porous body 80. In this case, the whole inner wall face of the sidewall opening part 68 can be wetted by lubricating liquid.

### <Fifth Embodiment>

Next, a fifth embodiment of the present invention will be described. Explanation of parts in common with those in the first embodiment is omitted and feature parts of the present embodiment will be mainly described below.

Fig. 14 is a schematic view (a side sectional view) showing a configuration example of the vicinity of a distal end of an insertion assisting tool 60D according to the fifth embodiment.

In the fifth embodiment, as shown in Fig. 14, such a configuration is adopted that corner portions 82 and 84 (an outer edge portion 82 and an inner edge portion 84) of the distal end edge part 68a of the sidewall opening part 68 of the tube main body 64 are subjected to a rounding work, respectively. According to such a configuration, when the insertion part 12 is inserted into a body cavity while being received and supported by the distal end edge part 68a of the sidewall opening part 68, lubricating liquid is effectively prevented from being scraped off due to contact of the insertion part 12 with the corner portion 82 or 84 of the distal end edge part 68a of the sidewall opening part 68.

### <Sixth Embodiment>

Next, a sixth embodiment of the present invention will be described. Explanation of parts in common with those in the first embodiment is omitted and feature parts of the present embodiment will be mainly described below.

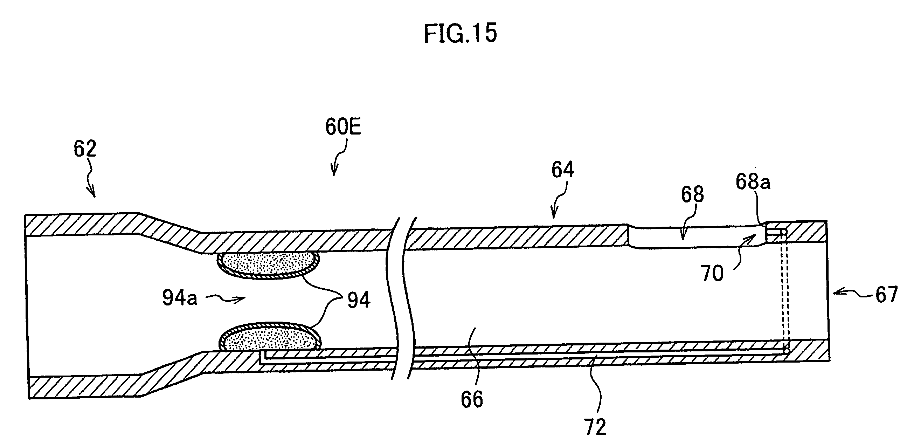

Fig. 15 is a schematic view (a side sectional view) showing a configuration example of an insertion assisting tool 60E according to the sixth embodiment.

In the sixth embodiment, as shown in Fig. 15, a balloon for lubricating liquid supply (hereinafter, simply called "balloon") 94 filled with lubricating liquid is provided inside the tube main body 64 on the distal end side of the tube main body 64. The balloon 94 is formed in a donut shape (annular shape) extending along a circumferential direction of the inner wall face of the tube main body 64, and lubricating liquid is filed in the balloon 94 (that is, a space part formed between the inner circumferential face of the tube main body 64 and the balloon 94). Further, a proximal end portion of the lubricating liquid supply passage 72 is opened in the balloon 94.

A central opening part 94a of the balloon 94 constitutes an insertion port for the insertion part 12, an opening diameter of the central opening part 94a is a diameter smaller than an outer diameter of the insertion part 12 before the insertion part 12 is inserted into the central opening part 94a. Thereby, when the insertion part 12 is inserted into the central opening part 94a, the balloon 94 is applied with a predetermined pressure from the insertion part 12 so that lubricating liquid filled in the balloon 94 can be supplied from the supply port 70 to the distal end edge part 68a of the sidewall opening part 68 via the lubricating liquid supply passage 72.

According to the sixth embodiment, when the insertion part 12 is inserted into the insertion assisting tool 60E, lubricating liquid is automatically supplied to the sidewall opening part 68, so that operation for supplying lubricating liquid is not required and operation burden of an operator can be reduced.

### <Seventh Embodiment>

Next, a seventh embodiment of the present invention will be described. Explanation of parts in common with those in the first embodiment is omitted and feature parts of the present embodiment will be mainly described below.

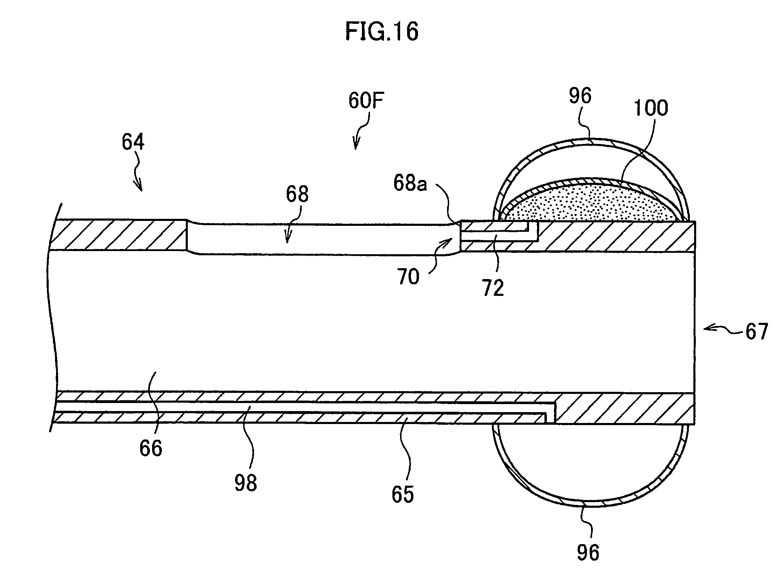

Fig. 16 is a schematic view (a side sectional view) showing a configuration example of an insertion assisting tool 60F according to the seventh embodiment.

In the seventh embodiment, as shown in Fig. 16, a fixing balloon 96 for fixing the insertion assisting tool 60F to a luminal wall (for example, an inner wall of the duodenum) is attached to an outer circumferential face of the sidewall part 65 of the tube main body 64 positioned beyond the sidewall opening part 68 on a distal end side of the tube main body 64. The fixing balloon 96 is in communication with a fluid channel 98 provided in the sidewall part 65 of the tube main body 64 so as to extend in an axial direction of the tube main body 64. The other end portion of the fluid channel 98 is connected to a balloon control device (not shown). By supplying fluid such as air into the fixing balloon 96 from the balloon control device via the fluid channel 98, the fixing balloon 96 is inflated annularly around the tube main body 64 so that the insertion assisting tool 60F is fixed to the luminal wall. Further, by discharging fluid in the fixing balloon 96 from the balloon control device via the fluid channel 98, fixation of the insertion assisting tool 60F to the luminal wall is released so that the insertion assisting tool 60F can be moved forward and backward.

A bag-shaped member 100 filled with lubricating liquid is provided inside the fixing balloon 96. One end of the lubricating liquid supply passage 72 is connected to the supply port 70 of the distal end edge part 68a of the sidewall opening part 68 and the other end of the lubricating liquid supply passage 72 is opened into the bag-shaped member 100. Thereby, when fluid is supplied from the balloon control device into the fixing balloon 96, an internal pressure of the fixing balloon 96 is increased so that the fixing balloon 96 is inflated outward and the bag-shaped member 100 is pressed inward. As a result, a filling agent in the bag-shaped member 100 is supplied so as to be pushed out from the supply port 70 via the lubricating liquid supply passage 72. Incidentally, such setting is adopted that allocation to a force for pressing the fixing balloon 96 outward and a force for pressing the bag-shaped member 100 inward occurs due to a relationship between the thickness of the fixing balloon 96 and the thickness of the bag-shaped member 100 or the like when the fixing balloon 96 inflates (starts inflating).

According to the seventh embodiment, when the insertion assisting tool 60F is fixed to a luminal wall by inflating the fixing balloon 96, lubricating liquid is automatically supplied to the sidewall opening part 68, so that operation for supplying lubricating liquid is made unnecessary and operation burden of an operator can be reduced.

### <Eighth Embodiment>

Next, an eighth embodiment of the present invention will be described. Explanation of parts in common with those in the first embodiment is omitted and feature parts of the present embodiment will be mainly described below.

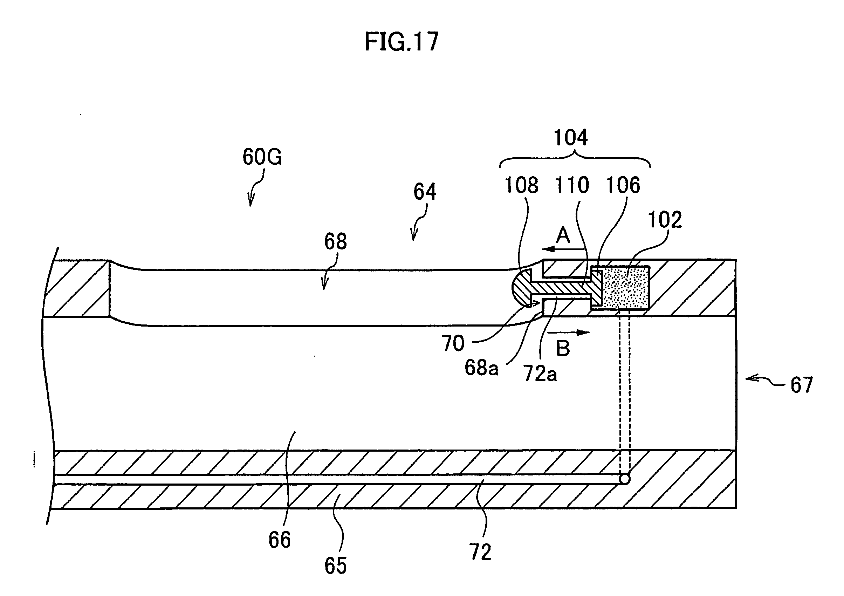

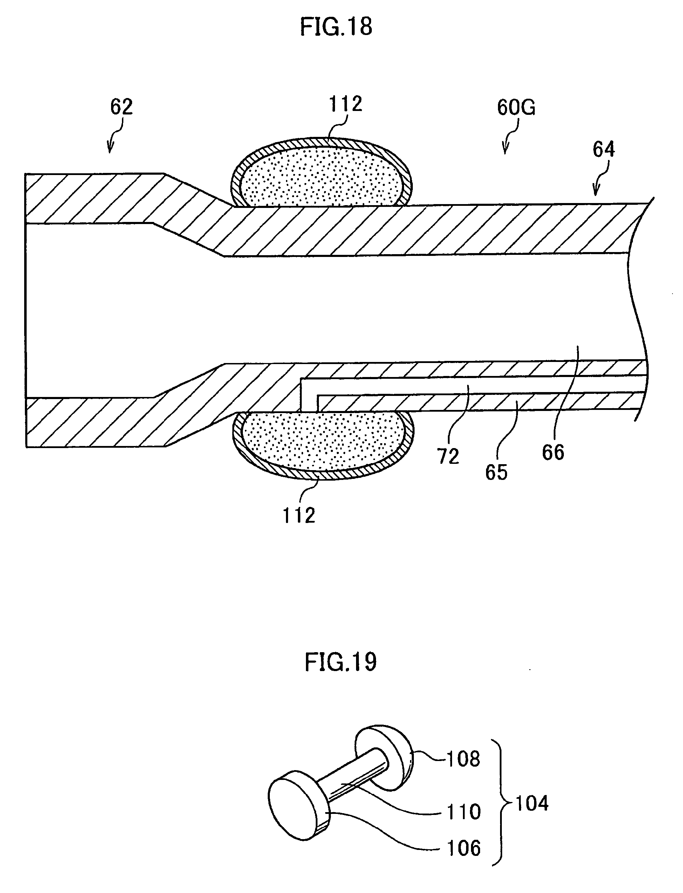

Fig. 17 and Fig. 18 are schematic views (side sectional views) showing a configuration example of an insertion assisting tool 60G according to the eighth embodiment, Fig. 17 showing a configuration on a distal end side of the insertion assisting tool 60G and Fig. 18 showing a configuration on a proximal end side of the insertion assisting tool 60G. Further, Fig. 19 is a perspective view showing a piece member provided in the insertion assisting tool 60G.

In the eighth embodiment, as shown in Fig. 17, a space part 102 constituting a portion of the lubricating liquid supply passage 72 is formed in the sidewall part 65 of the tube main body 64 positioned on a distal end side of the tube main body 64 beyond the sidewall opening part 68. A piece member 104 is provided between the space part 102 and the sidewall opening part 68. The piece member 104 is disposed in such a state that a disk-shaped proximal end part 106 of the piece member 104 is fitted in the space part 102 and a semi-spherical distal end part 108 of the piece member 104 is exposed in the sidewall opening part 68, and the proximal end part 106 and the distal end part 108 are connected to each other via a shaft part 110.

Further, as shown in Fig. 18, a bag-shaped member 112 filled with lubricating liquid in a pressurized state is provided on an outer circumferential face of the sidewall part 65 of the tube main body 64 on a proximal end side of the insertion assisting tool 60G. The proximal end portion of the lubricating liquid supply passage 72 is opened in the bag-shaped member 112. In the eighth embodiment, such a configuration is adopted that the bag-shape member 112 is formed over the whole circumferential direction of the outer circumferential face of the sidewall part 65, but the present invention is not limited to this configuration and the bag-shape member 112 may be formed only partially in the circumferential direction of the outer circumferential face of the sidewall part 65.

With such a configuration, the piece member 104 is biased in an A direction by lubricating liquid supplied from the bag-shaped member 112 to the space part 102 through the lubricating liquid supply passage 72 before the distal end of the insertion part 12 is fed from the sidewall opening part 68, so that the space part 102 and the supply port 70 are put in a non-communication state and lubricating liquid is not supplied to the supply port 70.

On the other hand, when the distal end of the insertion part 12 is fed from the sidewall opening part 68, the insertion part 12 abuts on the distal end part 108 of the piece member 104 so that the piece member 104 is moved in a B direction by a predetermined amount. Thereby, the space part 102 and the supply port 70 are put in communication with each other, so that lubricating liquid is supplied to the supply port 70.

According to the eighth embodiment, when the distal end of the insertion part 12 is fed from the sidewall opening part 68, lubricating liquid is automatically supplied to the sidewall opening part 68, so that operation for supplying lubricating liquid is not make unnecessary and operation burden of an operator can be reduced.

As described above, though the insertion assisting tool for an endoscope of the present invention has been described in detail, the present invention is not limited to the above embodiments, and can be improved or modified variously without departing from the scope of the present invention, of course.

## Claims

1. An insertion assisting tool (60) for an endoscope (10) comprising:
a tubular body having a passage (66) through which an insertion part (12) of an endoscope (10) is inserted, and
a sidewall opening part (68) which is provided in a sidewall part of the tubular body on a distal end side of the tubular body and through which a distal end part of the insertion part can be fed, the sidewall opening part (68) having an edge part (68a) receiving and supporting the insertion part (12) when the distal end part of the insertion part (12) is fed from the sidewall opening part (68) to be guided into a body cavity, **characterized by** comprising:
a supply port (70) formed at the edge part of the sidewall opening part (68); and
a lubricating liquid supply passage (72) which is in communication with the supply port (70) to supply lubricating liquid to the supply port (70).

2. The insertion assisting tool (60) for an endoscope (10) according to claim 1, wherein the edge part (68a) of the sidewall opening part (68) is positioned on a distal end side of the sidewall opening part (68).

3. The insertion assisting tool (60) for an endoscope (10) according to claim 1 or 2, wherein the lubricating liquid supply passage (72) is provided inside the sidewall part of the tubular body.

4. The insertion assisting tool (60) for an endoscope (10) according to any one of claims 1 to 3, wherein the supply port (70) is composed of a plurality of supply port elements arranged at the edge portion (68a) of the sidewall opening part (68).

5. The insertion assisting tool (60) for an endoscope (10) according to any one of claims 1 to 4, wherein a porous body (80) is provided at the edge part (68a) of the sidewall opening part (68) and the supply port (70) is composed of fine holes formed on a surface of the porous body (80).

6. The insertion assisting tool (60) for an endoscope (10) according to any one of claims 1 to 5, wherein a corner portion of the edge part (68a) of the sidewall opening part (68) is chamfered.

## Patentansprüche

1. Einführungsunterstützungswerkzeug (60) für ein Endoskop (10), umfassend:
einen schlauchförmigen Körper mit einem Durchgang (66) durch den ein Einführungsteil (12) eines Endoskops (10) eingeführt wird, und
ein Seitenwandöffnungsteil (68), das in einem Seitenwandteil des schlauchförmigen Körpers an einer distalen Endseite des schlauchförmigen Körpers vorgesehen ist und durch welches ein Distalendeteil des Einführungsteils zugeführt werden kann, wobei das Seitenwandöffnungsteil (68) einen Kantenteil (68a) besitzt, der das Einführungsteil (12) aufnimmt und unterstützt, wenn das distale Endteil des Einführungsteils (12) von dem Seitenwandöffnungsteil (68) aus zugeführt wird, um in eine Körperkavität geleitet zu werden,
**dadurch gekennzeichnet, dass** es umfasst:
einen Speiseanschluss (70), der in dem Kantenteil des Seitenwandöffnungsteils (68) ausgebildet ist; und
einen Gleitflüssigkeitsspeisedurchgang (72), der mit dem Speiseanschluss (70) in Kommunikation steht, um den Speiseanschluss (70) mit Gleitflüssigkeit zu speisen.

2. Einführungsunterstützungswerkzeug (60) für ein Endoskop (10) nach Anspruch 1, wobei das Kantenteil (68a) des Seitenwandöffnungsteils (68) an einer distalen Endseite des Seitenwandöffnungsteils (68) positioniert ist.

3. Einführungsunterstützungswerkzeug (60) für ein Endoskop (10) nach Anspruch 1 oder 2, wobei der Gleitflüssigkeitsspeisedurchgang (72) im Innern des Seitenwandteils des schlauchförmigen Körpers vorgesehen ist.

4. Einführungsunterstützungswerkzeug (60) für ein Endoskop (10) nach einem der Ansprüche 1 bis 3, wobei der Speiseanschluss (70) aus einer Vielzahl von Speiseanschlusselementen an dem Kantenabschnitt (68a) des Seitenwandöffnungsteils (68) aufgebaut ist.

5. Einführungsunterstützungswerkzeug (60) für ein Endoskop (10) nach einem der Ansprüche 1 bis 4, wobei ein poröser Körper (80) am Kantenteil (68a) des Seitenwandöffnungsteils (68) vorgesehen ist und der Speiseanschluss (70) aus feinen, auf einer Oberfläche des porösen Körpers (80) ausgebildeten Löchern aufgebaut ist.

6. Einführungsunterstützungswerkzeug (60) für ein Endoskop (10) nach einem der Ansprüche 1 bis 5, wobei ein Eckabschnitt des Kantenteils (68a) des Seitenwandöffnungsgteils (68) abgeschrägt ist.

## Revendications

1. Outil d'aide à l'insertion (60) pour un endoscope (10), comprenant :
un corps tubulaire présentant un passage (66) à travers lequel une partie d'insertion (12) d'un endoscope (10) est insérée, et
une partie d'ouverture de paroi latérale (68) prévue dans une partie de paroi latérale du corps tubulaire sur un côté d'extrémité distale du corps tubulaire et à travers laquelle une partie d'extrémité distale de la partie d'insertion peut être introduite, la partie d'ouverture de paroi latérale (68) présentant une partie de bord (68a) recevant et supportant la partie d'insertion (12) lorsque la partie d'extrémité distale de la partie d'insertion (12) est introduite à partir de la partie d'ouverture de paroi latérale (68) devant être guidée dans une cavité corporelle,
**caractérisé en ce qu'**il comprend :
un orifice d'alimentation (70) formé au niveau de la partie de bord de la partie d'ouverture de paroi latérale (68), et
un passage d'alimentation pour liquide lubrifiant (72) qui est en communication avec l'orifice d'alimentation (70) afin de fournir le liquide lubrifiant à l'orifice d'alimentation (70).

2. Outil d'aide à l'insertion (60) pour un endoscope (10) selon la revendication 1, dans lequel la partie de bord (68a) de la partie d'ouverture de paroi latérale (68) est positionnée sur un côté d'extrémité distale de la partie d'ouverture de paroi latérale (68).

3. Outil d'aide à l'insertion (60) pour un endoscope (10) selon la revendication 1 ou 2, dans lequel le passage d'alimentation pour liquide lubrifiant (72) est prévu à l'intérieur de la partie de paroi latérale du corps tubulaire.

4. Outil d'aide à l'insertion (60) pour un endoscope (10) selon l'une quelconque des revendications 1 à 3, dans lequel l'orifice d'alimentation (70) est composé d'une pluralité d'éléments d'orifice d'alimentation agencés au niveau de la portion de bord (68a) de la partie d'ouverture de paroi latérale (68).

5. Outil d'aide à l'insertion (60) pour un endoscope (10) selon l'une quelconque des revendications 1 à 4, dans lequel un corps poreux (80) est prévu au niveau de la partie de bord (68a) de la partie d'ouverture de paroi latérale (68), et l'orifice d'alimentation (70) est composé de trous fins formés sur une surface du corps poreux (80).

6. Outil d'aide à l'insertion (60) pour un endoscope (10) selon l'une quelconque des revendications 1 à 5, dans lequel une portion de coin de la partie de bord (68a) de la partie d'ouverture de paroi latérale (68) est chanfreinée.
